# EUROPÄISCHE PATENTSCHRIFT

(11) **EP 3 229 764 B1**
(45) Veröffentlichungstag und Bekanntmachung des Hinweises auf die Patenterteilung: **18.09.2019**
(21) Anmeldenummer: 15790099.4
(22) Anmeldetag: 02.11.2015
(51) Int. Cl.: A61K 8/34, A61Q 5/12, A61Q 13/00, A61Q 15/00, A61K 8/06

(54) **MITTEL UND VERFAHREN ZUR KOSMETISCHEN BEHANDLUNG**
AGENT AND METHOD FOR COSMETIC TREATMENT
AGENT ET PROCÉDÉ DE TRAITEMENT COSMÉTIQUE

(30) Priorität: 10.12.2014 DE 102014225434
(43) Veröffentlichungstag der Anmeldung: 18.10.2017
(73) Patentinhaber: Henkel AG & Co. KGaA, 40589 Düsseldorf (DE)
(72) Erfinder: FÖRSTER, Thomas, 40591 Düsseldorf (DE); BAYERSDÖRFER, Rolf, 22589 Hamburg (DE); KNAPPE, Thorsten, 22869 Schenefeld (DE)
(86) Internationale Anmeldenummer: PCT/EP2015/075394
(87) Internationale Veröffentlichungsnummer: WO 2016/091470

(56) Entgegenhaltungen:
- WO-A2-02/12090
- DE-A1-102011 086 019
- DE-A1-102011 086 923
- GB-A- 1 300 399
- GB-A- 1 410 012
- GB-A- 1 462 632
- JP-A- 2007 319 234
- US-A- 3 372 840
- US-A- 4 067 480
- US-A- 5 800 805
- US-A1- 2002 074 349
- US-A1- 2002 079 377
- US-A1- 2004 065 683
- US-A1- 2013 018 333

## Beschreibung

Die Anmeldung betrifft das technische Fachgebiet der Behandlung menschlicher Haut und menschlicher Haare. Gegenstand der Anmeldung sind spezifische kosmetische Formulierungen, welche sich zur Applikation auf Haut und Haar mittels eines Entspannungsverdampfungsverfahrens eignen. Darüber hinaus sind die Verwendung dieser kosmetischen Formulierungen in Vorrichtungen zur Entspannungsverdampfung und Verfahren zur Haarbehandlung Gegenstand der vorliegenden Anmeldung
Für viele Menschen ist die Parfümierung von Haut oder Haar ein wesentlicher Aspekt ihrer täglichen Körperpflege.

Im Bereich der Parfümierung hat die Sprühapplikation riechstoffhaltiger Zubereitungen eine sehr große Bedeutung, wobei die Zubereitungen in der Regel als Pumpsprays oder Aerosolsprays appliziert werden. Hierzu werden die riechstoffhaltigen Zubereitungen in einer Abgabevorrichtung konfektioniert, aus denen sie entweder mittels mechanischer Krafteinwirkung oder mit Hilfe eines Treibmittels über ein Ventil versprüht werden. Beide Methoden haben offensichtliche Nachteile. Während Pumpsprays sich in der Regel nicht für eine lang anhaltende gleichmäßige Sprühapplikation riechstoffhaltiger Zubereitungen eignen, basieren Aersolsprays auf dem Einsatz von Treibmitteln oder Treibgasen, die einerseits keine kosmetische Wirkung entfalten und von denen andererseits bei unsachgemäßer Handhabung eine Gefährdung der Verbraucher ausgehen kann. Vor diesem Hintergrund besteht ein Bedarf nach alternativen Wegen zur Zerstäubung riechstoffhaltiger Zubereitungen, wie in z.B. US 5 800 805 A beschrieben. Als ein solches alternatives Sprühverfahren hat sich die Entspannungsverdampfung erwiesen. Bei diesem Verfahren, das beispielsweise in der internationalen Patentanmeldung WO 2001/83071 A1 (Henkel) beschrieben wird, wird eine flüssige oder pastöse lösungsmittelhaltige Zusammensetzung in einem abgeschlossenen Raum auf eine Temperatur erhitzt, die über dem Siedepunkt des Lösungsmittels liegt, wodurch in der Zusammensetzung ein Überdruck erzeugt wird. Bei Entspannung (Drosselung) des Drucks verdampft die Flüssigkeit und kann nachfolgend beispielsweise mittels einer geeigneten Düse zerstäubt werden.

Auch wenn die Entspannungsverdampfung also grundsätzlich für die Sprühapplikation riechstoffhaltiger Zubereitungen geeignet ist, so kann gleichzeitig doch nicht jede haarkosmetische Zubereitung mittels eines Entspannungsverdampfungsverfahrens zerstäubt werden. Dies liegt einerseits an der für die Entspannungsverdampfung notwendigen Erhitzung der kosmetischen Zubereitung, andererseits an den Spezifika der durch Entspannungsverdampfung erzeugten Sprühnebel, beispielsweise der erzeugten Tröpfchengröße und Tröpfchendichte im Sprühnebel.

Aufgabe der vorliegenden Erfindung war es daher, spezifische riechstoffhaltige Zubereitungen zur Parfümierung von Haut oder Haar zur Verfügung zu stellen, welche sich aufgrund ihrer chemischen und physikalischen Eigenschaften zur zielgerichteten Sprühapplikation mittels einer Vorrichtung zur Entspannungsverdampfung eignen. Weiterhin sollen die Zubereitungen geeignet sein, nach einer Applikation mittels eines Entspannungsverfahrens eine gute Parfümierung zu realisieren. Es hat sich gezeigt, dass zur Lösung dieser Aufgabe aus der Vielzahl bekannter riechstoffhaltiger Zubereitungen insbesondere alkoholische Riechstoffzubereitungen geeignet sind.

Ein erster Gegenstand der vorliegenden Erfindung ist somit ein kosmetisches Produkt, umfassend
a) eine kosmetische Zubereitung, enthaltend bezogen auf ihr Gesamtgewicht
   a1) 20 bis 99 Gew.-% Ethanol;
   a2) 0,05 bis 20 Gew.-% Riechstoff;
b) eine Vorrichtung zur Entspannungsverdampfung der kosmetischen Zubereitung a).

Die kosmetische Zubereitung a) ist vorzugsweise flüssig. Die kosmetische Zubereitung a) kann als Lösung oder Dispersion, beispielsweise als Emulsion oder Suspension vorliegen. Bevorzugte kosmetische Zubereitungen a) liegen in Form einer Lösung oder einer Suspension vor.

Die erfindungsgemäße kosmetische Zubereitung enthält als ersten wesentlichen Bestandteil 20 bis 99 Gew.-% Ethanol a1). Bevorzugte kosmetische Produkte sind dadurch gekennzeichnet, dass der Gewichtsanteil des Ethanols a1) am Gesamtgewicht der kosmetischen Zubereitung a) 30 bis 90 Gew.-%, vorzugsweise 40 bis 80 Gew.-% beträgt. Entsprechende Mittel zeichnen sich durch eine gute kosmetische Wirkung bei gleichzeitig guter Applizierbarkeit aus.

Das Ethanol a1) kann als alleiniges Lösungsmittel oder in Kombination mit einem oder mehreren weiteren polaren Lösungsmitteln eingesetzt werden. Zur Verbesserung der Anwendungseigenschaften erfindungsgemäßer kosmetischer Zubereitungen bei gleichzeitiger Minimierung der thermischen Belastung etwaiger Wirk- oder Hilfsstoffe im Verlauf des Entspannungsverdampfungsverfahrens, hat es sich als vorteilhaft erwiesen, polare Lösungsmittel einzusetzen, welche eine Siedetemperatur (20°C, 1013 mbar) zwischen 50 und 110°C, vorzugsweise zwischen 70 und 105°C aufweisen. Als besonders geeignet hat sich Wasser erwiesen, welches aus diesem Grund als weiteres polares Lösungsmittel bevorzugt wird.

Bevorzugte kosmetische Zubereitungen a) sind dadurch, dass der Gewichtsanteil von Wasser und Ethanol an ihrem Gesamtgewicht mindestens 60 Gew.-%, bevorzugt mindestens 80 Gew.-%, besonders bevorzugt mindestens 90 Gew.-% und insbesondere mindestens 95 Gew.-% beträgt.

In den Fällen, in denen die kosmetische Zubereitung a Ethanol und Wasser umfasst, ist es bevorzugt, wenn das und Gewichtsverhältnis von Ethanol zu Wasser 50:1 bis 1:2, bevorzugt 40:1 bis 1:1 und insbesondere 30:1 bis 10:1 beträgt.

Ein zweiter wesentlicher Bestandteil erfindungsgemäßer kosmetischer Zusammensetzungen ist der Riechstoff a2). Bezüglich ihrer Anwendungseigenschaften haben sich kosmetische Zubereitungen a) als vorteilhaft erwiesen, bei denen der Gewichtsanteil des Riechstoffs am Gesamtgewicht der kosmetischen Zubereitung a) 0,1 bis 15 Gew.-% und insbesondere 0,5 bis 10 Gew.-% beträgt.

Der Begriff der "Riechstoffe" umfasst natürliche wie synthetische Substanzen, die olfaktorisch wahrgenommen werden können und einen Geruch entwickeln. Bei dem Riechstoff a) kann es sich um eine einzelne chemische Substanz aber auch um ein Substanzgemisch handeln, wobei der Einsatz von Substanzgemischen aufgrund der komplexeren Duftnote erfindungsgemäß bevorzugt ist.

Um wahrnehmbar zu sein, muß ein Riechstoff flüchtig sein, wobei neben der Natur der funktionellen Gruppen und der Struktur der chemischen Verbindung auch die Molmasse eine wichtige Rolle spielt. So besitzen die meisten Riechstoffe Molmassen bis etwa 200 Dalton, während Molmassen von 300 Dalton und darüber eher eine Ausnahme darstellen. Aufgrund der unterschiedlichen Flüchtigkeit von Riechstoffen verändert sich der Geruch eines aus mehreren Riechstoffen zusammengesetzten Parfüms bzw. Duftstoffs während des Verdampfens, wobei man die Geruchseindrücke in "Kopfnote" (top note), "Herz-bzw. Mittelnote" (middle note bzw. body) sowie "Basisnote" (end note bzw. dry out) unterteilt. Da die Geruchswahrnehmung zu einem großen Teil auch auf der Geruchsintensität beruht, besteht die Kopfnote eines Parfüms bzw. Duftstoffs nicht allein aus leichtflüchtigen Verbindungen, während die Basisnote zum größten Teil aus weniger flüchtigen, d.h. haftfesten Riechstoffen besteht.

Im Rahmen der Applikation von Riechstoffen mittels Entspannungsverdampfung kommt dem Siedepunkt der Riechstoffe eine gewisse Bedeutung zu. Als für die Anwendung und kosmetische Wirkung der kosmetischen Zubereitung a) vorteilhaft hat es sich erwiesen, wenn der Riechstoff a2) einen Siedepunkt oberhalb 80°C, vorzugsweise oberhalb 120°C und insbesondere oberhalb 160°C aufweist. Vorzugsweise weisen wenigstens 60 Gew.-%, bevorzugt mindestens 80 Gew.-% und insbesondere mindestens 90 Gew.-% des Riechstoffs a2) einen Siedepunkt oberhalb 80°C, vorzugsweise oberhalb 120°C und insbesondere oberhalb 160°C auf.

Aus den zuvor genannten Gründen sind insbesondere die höhersiedenden bzw. festen Riechstoffe natürlichen oder synthetischen Ursprungs im Rahmen der vorliegenden Erfindung bevorzugt. Zu diesen Substanzen zählen beispielsweise Ambrettolid, α-Amylzimtaldehyd, Anethol, Anisaldehyd, Anisalkohol, Anisol, Anthranilsäuremethylester, Acetophenon, Benzylaceton, Benzaldehyd, Benzoesäureethylester, Benzophenon, Benzylakohol, Benzylacetat, Benzylbenzoat, Benzylformiat, Benzylvalerianat, Borneol, Bornylacetat, α-Bromstyrol, n-Decylaldehyd, n-Dodecylaldehyd, Eugenol, Eugenolmethylether, Eukalyptol, Farnesol, Fenchon, Fenchylacetat, Geranylacetat, Geranylformiat, Heliotropin, Heptincarbonsäuremethylester, Heptaldehyd, Hydrochinon-Dimethylether, Hydroxyzimtaldehyd, Hydroxyzimtalkohol, Indol, Iron, Isoeugenol, Isoeugenolmethylether, Isosafrol, Jasmon, Kampfer, Karvakrol, Karvon, p-Kresolmethylether, Cumarin, p-Methoxyacetophenon, Methyl-n-amylketon, Methylanthranilsäuremethylester, p-Methylacetophenon, Methylchavikol, p-Methylchinolin, Methyl-β-naphthylketon, Methyl-n-nonylacetaldehyd, Methyl-n-nonylketon, Muskon, β-Naphtholethylether, β-Naphtholmethylether, Nerol, Nitrobenzol, n-Nonylaldehyd, Nonylakohol, n-Octylaldehyd, p-Oxy-Acetophenon, Pentadekanolid, β-Phenylethylakohol, Phenylacetaldehyd-Dimethyacetal, Phenylessigsäure, Pulegon, Safrol, Salicylsäureisoamylester, Salicylsäuremethylester, Salicylsäurehexylester, Salicylsäurecyclohexylester, Santalol, Skatol, Terpineol, Thymen, Thymol, γ-Undelacton, Vanilin, Veratrumaldehyd, Zimtaldehyd, Zimatalkohol, Zimtsäure, Zimtsäureethylester und Zimtsäurebenzylester.

Alternativ zu den höhersiedenden bzw. festen Riechstoffen oder in Kombination mit diesen, werden als Riechstoffe a2) weiterhin bevorzugt die haftfesten Riechstoffe eingesetzt. Zur Gruppe dieser Riechstoffe zählen u.a. die ätherischen Öle wie Angelikawurzelöl, Anisöl, Arnikablütenöl, Basilikumöl, Bayöl, Bergamottöl, Champacablütenöl, Edeltannenöl, Edeltannenzapfenöl, Elemiöl, Eukalyptusöl, Fenchelöl, Fichtennandelöl, Galbanumöl, Geraniumöl, Gingergrasöl, Guajakholzöl, Gurjunbalsamöl, Helichrysumöl, Ho-Öl, Ingweröl, Irisöl, Kajeputöl, Kalmusöl, Kamillenöl, Kampferöl, Kanagaöl, Kardamomenöl, Kassiaöl, Kiefernnadelöl, Kopaïvabalsamöl, Korianderöl, Krauseminzeöl, Kümmelöl, Kuminöl, Lavendelöl, Lemongrasöl, Limetteöl, Mandarinenöl, Melissenöl, Moschuskörneröl, Myrrhenöl, Nelkenöl, Neroliöl, Niaouliöl, Olibanumöl, Orangenöl, Origanumöl, Palmarosaöl, Patschuliöl, Perubalsamöl, Petitgrainöl, Pfefferöl, Pfefferminzöl, Pimentöl, Pine-Öl, Rosenöl, Rosmarinöl, Sandelholzöl, Sellerieöl, Spiköl, Sternanisöl, Terpentinöl, Thujaöl, Thymianöl, Verbenaöl, Vetiveröl, Wacholderbeeröl, Wermutöl, Wintergrünöl, Ylang-Ylang-Öl, Ysop-Öl, Zimtöl, Zimtblätteröl, Zitronellöl, Zitronenöl sowie Zypressenöl.

Die erfindungsgemäßen kosmetischen Produkte umfassen neben der kosmetischen Zubereitung a) weiterhin eine Vorrichtung zur Entspannungsverdampfung. Der Ausdruck "Entspannungsverdampfung" bezeichnet im Rahmen der vorliegenden Anmeldung das Entstehen von Dampf bei Absenken des Druckes in einem mit Flüssigkeit befüllten, unter Überdruck (zur Umgebung) stehenden geschlossenen Raum. Ein entsprechender Überdruck lässt sich beispielsweise erzeugen, indem eine Menge der kosmetischen Zubereitung a) in einem abgeschlossenen Raum auf eine Temperatur T₁ erhitzt wird. In dem geschlossenen Raum hat die Flüssigkeit bei gegebener Temperatur T₁ einen Druck Sättigungsdruck p₁. Wird der geschlossenen Raum beispielsweise mittels eines Ventils zu einem nicht unter Überdruck stehenden Relaxationsraum mit dem Druck p₀ < p₁ geöffnet, so sinkt der Druck in dem zuvor geschlossenen Raum ab und im Rahmen der Ausbreitung des neuen Druckniveaus verdampft die kosmetische Zubereitung a), bzw. das in der kosmetischen Zubereitung enthaltene Lösungsmittel oder Teile dieses Lösungsmittels. Der entstehende Dampf oder Sprühnebel kann für die Applikation spezifischer kosmetischer Zubereitungen genutzt werden.

Wird die kosmetische Zubereitung a) also ausgehend von Standardbedingungen (T₀ = 25°C, p₀ = 1,000 bar) in einem geschlossenen Raum erhitzt, so resultiert neben einer erhöhten Temperatur weiterhin ein erhöhter Druck der kosmetischen Zubereitung a). Dieser erhöhte Druck kann in einem Relaxationsraum auf einen Druck po, zum Beispiel den umgebenden Luftdruck (po = 1,000 bar), entlastet werden kann, wodurch mindestens teilweise eine Verdampfung der kosmetischen Zubereitung a) erreicht wird.

Bei der Entspannungsverdampfung handelt es sich mit anderen Worten um ein Verfahren, bei welchem die kosmetische Zubereitung a) in einem geschlossenen Behälter mittels einer Heizvorrichtung auf Temperaturen oberhalb der Umgebungstemperatur erhitzt wird, wobei in dem Behälter ein Druck oberhalb des Umgebungsdrucks entsteht, und die erhitzte und unter erhöhtem Druck stehende kosmetische Zubereitung a) anschließend aus dem Behälter in die Umgebung entspannt wird.

Bei einer Vorrichtung zur Entspannungsverdampfung handelt es sich demnach um eine Vorrichtung, welche einen Behälter und eine Heizvorrichtung umfasst und derart ausgestaltet ist, dass eine kosmetische Zubereitung a) in dem geschlossenen Behälter mittels der Heizvorrichtung auf Temperaturen oberhalb der Umgebungstemperatur in einer Weise erhitzt werden kann, dass in dem Behälter ein Druck oberhalb des Umgebungsdrucks entsteht und die erhitzte und unter erhöhtem Druck stehende kosmetische Zubereitung a) aus dem Behälter in die Umgebung entspannt werden kann.

Gleichzeitig mit oder nach der Druckentlastung kann die kosmetische Zubereitung a) einer Düse zugeführt werden, mittels derer beispielsweise Eigenschaften des durch die Entspannungsverdampfung erzeugten Dampfes bzw. Sprühnebels, insbesondere die Tröpfchengröße oder die Tröpfchendichte aber auch die Sprühweite und die Form des Sprühkegels beeinflusst werden können. Der Einsatz von Düsen, vorzugsweise Zerstäuberdüsen, ist daher bevorzugt. Der spezifische Düsentyp oder die spezifische Düsengestaltung wird in Abhängigkeit von den jeweiligen Sprühnebeleigenschaften gezielt festgelegt.

Zusammenfassend verfügt die erfindungsgemäße Vorrichtung zur Entspannungsverdampfung über
b1) einen mittels eines Ventils zu verschließenden und zu öffnenden Behälter b1), welcher den geschlossenen Innenraum definiert, in dem die kosmetische Zubereitung aufgenommen werden kann,
b2) eine Heizvorrichtung b2), welche es ermöglicht eine in dem Behälter b1) befindliche kosmetische Zubereitung zu erhitzen, und zwingend die Anwendung einer zusätzlichen Düse b3), welche eine Zerstäubung der aus dem Behälter entweichenden kosmetischen Zubereitung a) ermöglicht. Alternativ zu einem Ventil kann auch ein vergleichbar wirkendes Schließelement, welches eine zugehörige Öffnung im Behälter durch entsprechende Positionsänderung verschließen oder freigeben kann, zum Einsatz kommen

Offenbart ist also ein kosmetisches Produkt, umfassend
a) eine kosmetische Zubereitung, enthaltend bezogen auf ihr Gesamtgewicht
   a1) 20 bis 99 Gew.-% Ethanol;
   a2) 0,05 bis 20 Gew.-% Riechstoff;
b) eine Vorrichtung zur Entspannungsverdampfung der kosmetischen Zubereitung a), wobei die Vorrichtung zur Entspannungsverdampfung einen Behälter b1) und eine Heizvorrichtung b2 umfasst und derart ausgestaltet ist, dass
   - die kosmetische Zubereitung a) in den Innenraum des Behälters b1) aufgenommen werden kann,
   - der mit der kosmetischen Zubereitung a) wenigstens anteilsweise befüllte Innenraum des Behälters b1) verschlossen werden kann,
   - die kosmetische Zubereitung a) in dem geschlossenen Innenraum des Behälters b1) mittels der Heizvorrichtung b2) unter Druckerhöhung erhitzt werden kann.

Offenbart ist also ein kosmetisches Produkt, umfassend
a) eine kosmetische Zubereitung, enthaltend bezogen auf ihr Gesamtgewicht
   a1) 20 bis 99 Gew.-% Ethanol;
   a2) 0,05 bis 20 Gew.-% Riechstoff;
b) eine Vorrichtung zur Entspannungsverdampfung der kosmetischen Zubereitung a), umfassend
   b1) einen mittels eines Ventils zu verschließenden und zu öffnenden Behälter b1)
   b2) eine Heizvorrichtung, welche es ermöglicht ein in dem Behälter b1) befindliche kosmetische Zubereitung zu erhitzen
   b3) eine Düse b3), welche eine Zerstäubung der kosmetischen Zubereitung a) ermöglicht.

Offenbart ist also ein kosmetisches Produkt, umfassend
a) eine kosmetische Zubereitung, enthaltend bezogen auf ihr Gesamtgewicht
   a1) 20 bis 99 Gew.-% Ethanol;
   a2) 0,05 bis 20 Gew.-% Riechstoff;
b) eine Vorrichtung zur Entspannungsverdampfung der kosmetischen Zubereitung a), wobei die Vorrichtung zur Entspannungsverdampfung einen Behälter b1) und eine Heizvorrichtung b2 umfasst und derart ausgestaltet ist, dass
   - die kosmetische Zubereitung a) in den Innenraum des Behälters b1) aufgenommen werden kann,
   - der mit der kosmetischen Zubereitung a) wenigstens anteilsweise befüllte Innenraum des Behälters b1) verschlossen werden kann,
   - die kosmetische Zubereitung a) in dem geschlossenen Innenraum des Behälters b1) mittels der Heizvorrichtung b2) unter Druckerhöhung erhitzt werden kann,
   - die erhitzte kosmetische Zubereitung a) aus dem Innenraum des Behälters b1) unter Druckminderung in die Umgebung entspannt werden kann.

Der Behälter b1), in welchem die kosmetische Zubereitung erhitzt wird, ist in einer Weise ausgestaltet, die es ermöglicht, diesen Behälter während der Erhitzung der kosmetischen Zubereitung a) gegen die Umgebung vollständig abzuschließen und nach der Erhitzung, zur Ermöglichung Entspannungsverdampfung der kosmetischen Zubereitung a), zu öffnen. Dies kann beispielsweise durch ein Bauteil zur Durchflussregelung, insbesondere ein Ventil, gewährleistet werden.

Der Behälter b1), in welchem die kosmetische Zubereitung erhitzt wird, steht vorzugsweise in Kontakt mit einem weiteren Behälter, aus welchem die zur Entspannungsverdampfung vorgesehene Menge der kosmetischen Zubereitung vor der Erhitzung in den Behälter b1) überführt wird. Der Zugang zwischen diesem Vorratsbehälter und Behälter b1) ist dabei über eine entsprechende Vorrichtung, beispielsweise ein Ventil, zu öffnen und zu schließen. Dieser weitere Behälter ist vorzugsweise in Form eines Vorratsbehälters ausgestaltet, das heißt, er umfasst bevorzugt ein Vielfaches, beispielsweise mehr als das Zehnfache, vorzugsweise mehr als das Fünfzigfache, der für einen Verdampfungsvorgang notwendigen Menge der kosmetischen Zubereitung. Mit anderen Worten weist der weitere Behälter/Vorratsbehälter vorzugsweise ein Vielfaches, beispielsweise mehr als das Zehnfache Volumen, vorzugsweise mehr als das Zwanzigfache und insbesondere mehr als das Fünfzigfache Volumen des Behälters b1) auf.

Offenbart ist also ein kosmetisches Produkt, umfassend
a) eine kosmetische Zubereitung, enthaltend bezogen auf ihr Gesamtgewicht
   a1) 20 bis 99 Gew.-% Ethanol;
   a2) 0,05 bis 20 Gew.-% Riechstoff;
b) eine Vorrichtung zur Entspannungsverdampfung der kosmetischen Zubereitung a), umfassend
   b1) einen mittels eines Ventils zu verschließenden und zu öffnenden Behälter b1)
   b2) eine Heizvorrichtung, welche es ermöglicht eine in dem geschlossenen Behälter b1) befindliche kosmetische Zubereitung zu erhitzen
   b3) eine Düse b3), welche eine Zerstäubung der kosmetischen Zubereitung a) ermöglicht.
c) einen Vorratsbehälter für die kosmetische Zubereitung a), aus welchem die kosmetische Zubereitung a) in den Behälter b1) gelangen kann, wobei
   ∘ der Zugang zwischen Vorratsbehälter und Behälter b1) ein Bauteil zur Durchflussregelung aufweist, mittels dessen der Durchfluss der kosmetischen Zubereitung a) aus dem Vorratsbehälter in den Behälter b1) unterbrochen werden kann;
   ∘ der Vorratsbehälter mindestens das Zehnfache Volumen, vorzugsweise mindestens das Zwanzigfache und insbesondere mindestens das Fünfzigfache Volumen des Behälters b1) aufweist.

Der Vorratsbehälter ist kein Druckbehälter und die in dem Vorratsbehälter befindliche kosmetische Zusammensetzung befindet sich nicht unter Druck, mit anderen Worten entspricht der Druck im Inneren des Vorratsbehälters dem Umgebungsdruck (auch Luftdruck oder Atmosphärendruck). So umfassen entsprechende kosmetische Produkte beispielsweise keine Treibmittel. Auch verfügt das kosmetische Produkt über keine Pumpvorrichtung, die geeignet ist, die kosmetische Zubereitung ohne die Einwirkung der Vorrichtung zur Entspannungsverdampfung in die Umgebung freizusetzen oder zu versprühen.

Offenbart ist also ein kosmetisches Produkt, umfassend
a) eine kosmetische Zubereitung, enthaltend bezogen auf ihr Gesamtgewicht
   a1) 20 bis 99 Gew.-% Ethanol;
   a2) 0,05 bis 20 Gew.-% Riechstoff;
b) eine Vorrichtung zur Entspannungsverdampfung der kosmetischen Zubereitung a), umfassend
   b1) einen mittels eines Ventils zu verschließenden und zu öffnenden Behälter b1)
   b2) eine Heizvorrichtung, welche es ermöglicht eine in dem geschlossenen Behälter b1) befindliche kosmetische Zubereitung zu erhitzen
   b3) eine Düse b3), welche eine Zerstäubung der kosmetischen Zubereitung a) ermöglicht.
c) einen Vorratsbehälter für die kosmetische Zubereitung a), aus welchem die kosmetische Zubereitung a) in den Behälter b1) gelangen kann, wobei
   ∘ der Zugang zwischen Vorratsbehälter und Behälter b1) ein Bauteil zur Durchflussregelung aufweist, mittels dessen der Durchfluss der kosmetischen Zubereitung a) aus dem Vorratsbehälter in den Behälter b1) unterbrochen werden kann;
   ∘ der Vorratsbehälter mindestens das Zehnfache Volumen, vorzugsweise mindestens das Fünfzigfache Volumen des Behälters b1) aufweist;
   ∘ der Druck im Inneren des Vorratsbehälters dem Umgebungsdruck entspricht.

Offenbart ist also ein kosmetisches Produkt, umfassend
a) eine kosmetische Zubereitung, enthaltend bezogen auf ihr Gesamtgewicht
   a1) 20 bis 99 Gew.-% Ethanol;
   a2) 0,05 bis 20 Gew.-% Riechstoff;
b) eine Vorrichtung zur Entspannungsverdampfung der kosmetischen Zubereitung a), umfassend
   b1) einen mittels eines Ventils zu verschließenden und zu öffnenden Behälter b1)
   b2) eine Heizvorrichtung, welche es ermöglicht eine in dem geschlossenen Behälter b1) befindliche kosmetische Zubereitung zu erhitzen
   b3) eine Düse b3), welche eine Zerstäubung der kosmetischen Zubereitung a) ermöglicht.
c) einen Vorratsbehälter für die kosmetische Zubereitung a), aus welchem die kosmetische Zubereitung a) in den Behälter b1) gelangen kann, wobei
   ∘ der Zugang zwischen Vorratsbehälter und Behälter b1) ein Bauteil zur Durchflussregelung aufweist, mittels dessen der Durchfluss der kosmetischen Zubereitung a) aus dem Vorratsbehälter in den Behälter b1) unterbrochen werden kann;
   ∘ der Vorratsbehälter mindestens das Zehnfache Volumen, vorzugsweise mindestens das Fünfzigfache Volumen des Behälters b1) aufweist;
   ∘ der Druck im Inneren des Vorratsbehälters dem Umgebungsdruck entspricht und das kosmetische Produkt kein Treibmittel umfasst.

Ferner offenbart sind kosmetische Produkte, umfassend
a) eine kosmetische Zubereitung, enthaltend bezogen auf ihr Gesamtgewicht
   a1) 20 bis 99 Gew.-% Ethanol;
   a2) 0,05 bis 20 Gew.-% Riechstoff;
b) eine Vorrichtung zur Entspannungsverdampfung der kosmetischen Zubereitung a), umfassend
   b1) einen mittels eines Ventils zu verschließenden und zu öffnenden Behälter b1)
   b2) eine Heizvorrichtung, welche es ermöglicht eine in dem geschlossenen Behälter b1) befindliche kosmetische Zubereitung zu erhitzen
   b3) eine Düse b3), welche eine Zerstäubung der kosmetischen Zubereitung a) ermöglicht.
c) einen Vorratsbehälter für die kosmetische Zubereitung a), aus welchem die kosmetische Zubereitung a) in den Behälter b1) gelangen kann, wobei
   ∘ der Zugang zwischen Vorratsbehälter und Behälter b1) ein Bauteil zur Durchflussregelung aufweist, mittels dessen der Durchfluss der kosmetischen Zubereitung a) aus dem Vorratsbehälter in den Behälter b1) unterbrochen werden kann;
   ∘ der Vorratsbehälter mindestens das Zehnfache Volumen, vorzugsweise mindestens das Fünfzigfache Volumen des Behälters b1) aufweist;
   ∘ der Druck im Inneren des Vorratsbehälters dem Umgebungsdruck entspricht,
   wobei das kosmetische Produkt keine Pumpvorrichtung aufweist, die geeignet ist, die kosmetische Zubereitung a) ohne die Einwirkung der Vorrichtung zur Entspannungsverdampfung freizusetzen oder zu versprühen.

Ferner offenbart ist daher ein kosmetisches Produkt, umfassend
a) eine kosmetische Zubereitung, enthaltend bezogen auf ihr Gesamtgewicht
   a1) 20 bis 99 Gew.-% Ethanol;
   a2) 0,05 bis 20 Gew.-% Riechstoff;
b) eine Vorrichtung zur Entspannungsverdampfung der kosmetischen Zubereitung a), umfassend
   b1) einen mittels eines Ventils zu verschließenden und zu öffnenden Behälter b1)
   b2) eine Heizvorrichtung, welche es ermöglicht eine in dem geschlossenen Behälter b1) befindliche kosmetische Zubereitung zu erhitzen
   b3) eine Düse b3), welche eine Zerstäubung der kosmetischen Zubereitung a) ermöglicht.
c) einen Vorratsbehälter für die kosmetische Zubereitung a), aus welchem die kosmetische Zubereitung a) in den Behälter b1) gelangen kann, wobei
   ∘ der Zugang zwischen Vorratsbehälter und Behälter b1) ein Bauteil zur Durchflussregelung aufweist, mittels dessen der Durchfluss der kosmetischen Zubereitung a) aus dem Vorratsbehälter in den Behälter b1) unterbrochen werden kann;
   ∘ der Vorratsbehälter mindestens das Zehnfache Volumen, vorzugsweise mindestens das Fünfzigfache Volumen des Behälters b1) aufweist;
   ∘ der Druck im Inneren des Vorratsbehälters dem Umgebungsdruck entspricht und das kosmetische Produkt kein Treibmittel umfasst,
   wobei das kosmetische Produkt keine Pumpvorrichtung aufweist, die geeignet ist, die kosmetische Zubereitung a) ohne die Einwirkung der Vorrichtung zur Entspannungsverdampfung freizusetzen oder zu versprühen.

Neben den beiden zuvor beschriebenen Bestandteilen a1) und a2) können die erfindungsgemäßen kosmetischen Zubereitungen a) weitere Wirk- oder Hilfsstoffe enthalten, wobei insbesondere solche Wirk- oder Hilfsstoffe bevorzugt werden, welche die Herstellbarkeit, Applizierbarkeit und/oder kosmetische Wirkung erfindungsgemäßer kosmetischer Zubereitungen verbessern.

Zur Verbesserung der Herstellbarkeit, Applizierbarkeit und kosmetischen Wirkung enthält die kosmetische Zubereitung a) vorzugsweise nichtionisches Tensid a3), wobei besonders bevorzugte kosmetische Zubereitungen a) dadurch gekennzeichnet sind, dass sie bezogen auf ihr Gesamtgewicht 0,02 bis 4,0 Gew.-%, vorzugsweise 0,05 bis 2,0 Gew.-% und insbesondere 0,1 bis 1,0 Gew.-% nichtionisches Tensid a3) enthalten.

Bevorzugte nichtionische Tenside sind PEG-Derivate von hydriertem Ricinusöl, die z. B. unter der Bezeichnung PEG Hydrogenated Castor Oil erhältlich sind, z.B. PEG-30 Hydrogenated Castor Oil, PEG-33 Hydrogenated Castor Oil, PEG-35 Hydrogenated Castor Oil, PEG-36 Hydrogenated Castor Oil, PEG-40 Hydrogenated Castor Oil oder PEG-60 Hydrogenated Castor Oil. Erfindungsgemäß besonders bevorzugt sind nichtionische Tenside ausgewählt aus der Gruppe der PEG-Derivate von hydriertem Ricinusöl, besonders bevorzugt aus der Gruppe PEG-40 Hydrogenated Castor Oil und PEG-60 Hydrogenated Castor Oil, insbesondere PEG-40 Hydrogenated Castor Oil enthält.

Zur Verbesserung des Duftprofils enthalten bevorzugte kosmetische Zubereitungen a) filmbildendes Polymer a4). Als filmbildende Polymere a4) eignen sich permanent als auch temporär kationische, anionische, nichtionische oder amphotere Polymere. Diese filmbildenden Polymere können synthetischen oder natürlichen Ursprungs sein. Bevorzugte kosmetische Zubereitung a) enthalten bezogen auf ihr Gesamtgewicht 0,1 bis 20 Gew.-%, vorzugsweise 0,5 bis 15 Gew.-%, und insbesondere 1,0 bis 10 Gew.-% eines filmbildenden Polymers a4).

Beispiele für gebräuchliche filmbildende Polymere a3) sind Acrylamide/Ammonium Acrylate Copolymer, Acrylamides/DMAPA Acrylates/Methoxy PEG Methacrylate Copolymer, Acrylamidopropyltrimonium Chloride/Acrylamide Copolymer, Acrylamidopropyltrimonium Chloride/Acrylates Copolymer, Acrylates/Acetoacetoxyethyl Methacrylate Copolymer, Acrylates/Acrylamide Copolymer, Acrylates/Ammonium Methacrylate Copolymer, Acrylates/t-Butylacrylamide Copolymer, Acrylates Copolymer, Acrylates/C1-2 Succinates/Hydroxyacrylates Copolymer, Acrylates/Lauryl Acrylate/Stearyl Acrylate/Ethylamine Oxide Methacrylate Copolymer, Acrylates/Octylacrylamide Copolymer, Acrylates/Octylacrylamide/Diphenyl Amodimethicone Copolymer, Acrylates/Stearyl Acrylate/Ethylamine Oxide Methacrylate Copolymer, Acrylates/VA Copolymer, Acrylates/VP Copolymer, Adipic Acid/Diethylenetriamine Copolymer, Adipic Acid/Dimethylaminohydroxypropyl Diethylenetriamine Copolymer, Adipic Acid/Epoxypropyl Diethylenetriamine Copolymer, Adipic Acid/Isophthalic Acid/Neopentyl Glycol/Trimethylolpropane Copolymer, Allyl Stearate/VA Copolymer, Aminoethylacrylate Phosphate/Acrylates Copolymer, Aminoethylpropanediol-Acrylates/Acrylamide Copolymer, Aminoethylpropanediol-AMPD-Acrylates/Diacetoneacrylamide Copolymer, Ammonium VA/Acrylates Copolymer, AMPD-Acrylates/Diacetoneacrylamide Copolymer, AMP-Acrylates/Allyl Methacrylate Copolymer, AMP-Acrylates/C1-18 Alkyl Acrylates/c1-8 Alkyl Acrylamide Copolymer, AMP-Acrylates/Diacetoneacrylamide Copolymer, AMP-Acrylates/Dimethylaminoethylmethacrylate Copolymer, Bacillus/Rice Bran Extract/Soybean Extract Ferment Filtrate, Bis-Butyloxyamodimethicone/PEG-60 Copolymer, Butyl Acrylate/Ethylhexyl Methacrylate Copolymer, Butyl Acrylate/Hydroxypropyl Dimethicone Acrylate Copolymer, Butylated PVP, Butyl Ester of Ethylene/MA Copolymer, Butyl Ester of PVM/MA Copolymer, Calcium/Sodium PVM/MA Copolymer, Corn Starch/Acrylamide/ Sodium Acrylate Copolymer, Diethylene Glycolamine/Epichlorohydrin/Piperazine Copolymer, Dimethicone Crosspolymer, Diphenyl Amodimethicone, Ethyl Ester of PVM/MA Copolymer, Hydrolyzed Wheat Protein/PVP Crosspolymer, Isobutylene/Ethylmaleimide/ Hydroxyethylmaleimide Copolymer, Isobutylene/MA Copolymer, Isobutylmethacrylate/Bis-Hydroxypropyl Dimethicone Acrylate Copolymer, Isopropyl Ester of PVM/MA Copolymer, Lauryl Acrylate Crosspolymer, Lauryl Methacrylate/Glycol Dimethacrylate Crosspolymer, MEA-Sulfite, Methacrylic Acid/Sodium Acrylamidomethyl Propane Sulfonate Copolymer, Methacryloyl Ethyl Betaine/Acrylates Copolymer, Octylacrylamide/Acrylates/Butylaminoethyl Methacrylate Copolymer, PEG/PPG-25/25 Dimethicone/Acrylates Copolymer, PEG-8/SMDI Copolymer, Polyacrylamide, Polyacrylate-6, Polybeta-Alanine/Glutaric Acid Crosspolymer, Polybutylene Terephthalate, Polyester-1, Polyethylacrylate, Polyethylene Terephthalate, Polymethacryloyl Ethyl Betaine, Polypentaerythrityl Terephthalate, Polyperfluoroperhydrophenanthrene, Polyquaternium-1, Polyquaternium-2, Polyquaternium-4, Polyquaternium-5, Polyquaternium-6, Polyquaternium-7, Polyquaternium-8, Polyquaternium-9, Polyquaternium-10, Polyquaternium-11, Polyquaternium-12, Polyquaternium-13, Polyquaternium-14, Polyquaternium-15, Polyquaternium-16, Polyquaternium-17, Polyquaternium-18, Polyquaternium-19, Polyquaternium-20, Polyquaternium-22, Polyquaternium-24, Polyquaternium-27, Polyquaternium-28, Polyquaternium-29, Polyquaternium-30, Polyquaternium-31, Polyquaternium-32, Polyquaternium-33, Polyquaternium-34, Polyquaternium-35, Polyquaternium-36, Polyquaternium-39, Polyquaternium-45, Polyquaternium-46, Polyquaternium-47, Polyquaternium-48, Polyquaternium-49, Polyquaternium-50, Polyquaternium-55, Polyquaternium-56, Polysilicone-9, Polyurethane-1, Polyurethane-6, Polyurethane-10, Polyvinyl Acetate, Polyvinyl Butyral, Polyvinylcaprolactam, Polyvinylformamide, Polyvinyl Imidazolinium Acetate, Polyvinyl Methyl Ether, Potassium Butyl Ester of PVM/MA Copolymer, Potassium Ethyl Ester of PVM/MA Copolymer, PPG-70 Polyglyceryl-10 Ether, PPG-12/SMDI Copolymer, PPG-51/SMDI Copolymer, PPG-10 Sorbitol, PVM/MA Copolymer, PVP, PVP/VA/Itaconic Acid Copolymer, PVP/VA/Vinyl Propionate Copolymer, Rhizobian Gum, Rosin Acrylate, Shellac, Sodium Butyl Ester of PVM/MA Copolymer, Sodium Ethyl Ester of PVM/MA Copolymer, Sodium Polyacrylate, Sterculia Urens Gum, Terephthalic Acid/Isophthalic Acid/Sodium Isophthalic Acid Sulfonate/Glycol Copolymer, Trimethylolpropane Triacrylate, Trimethylsiloxysilylcarbamoyl Pullulan, VA/Crotonates Copolymer, VA/Crotonates/Methacryloxybenzophenone-1 Copolymer, VA/Crotonates/Vinyl Neodecanoate Copolymer, VA/Crotonates/Vinyl Propionate Copolymer, VA/DBM Copolymer, VA/Vinyl Butyl Benzoate/Crotonates Copolymer, Vinylamine/Vinyl Alcohol Copolymer, Vinyl Caprolactam/VP/Dimethylaminoethyl Methacrylate Copolymer, VP/Acrylates/Lauryl Methacrylate Copolymer, VP/Dimethylaminoethylmethacrylate Copolymer, VP/DMAPA Acrylates Copolymer, VP/Hexadecene Copolymer, VP/VA Copolymer, VP/Vinyl Caprolactam/DMAPA Acrylates Copolymer, Yeast Palmitate und Styrene/VP Copolymer.

Als weitere geeignete Wirk- oder Hilfsstoffe sind insbesondere zusätzliche Pflegestoffe zu nennen.

Als Pflegestoff kann das Mittel beispielsweise mindestens ein Proteinhydrolysat und/oder eines seiner Derivate enthalten. Proteinhydrolysate sind Produktgemische, die durch sauer, basisch oder enzymatisch katalysierten Abbau von Proteinen (Eiweißen) erhalten werden. Unter dem Begriff Proteinhydrolysate werden erfindungsgemäß auch Totalhydrolysate sowie einzelne Aminosäuren und deren Derivate sowie Gemische aus verschiedenen Aminosäuren verstanden. Das Molgewicht der erfindungsgemäß einsetzbaren Proteinhydrolysate liegt zwischen 75, dem Molgewicht für Glycin, und 200.000, bevorzugt beträgt das Molgewicht 75 bis 50.000 und ganz besonders bevorzugt 75 bis 20.000 Dalton.

Als Pflegestoff kann das erfindungsgemäße Mittel weiterhin mindestens ein Vitamin, ein Provitamin, eine Vitaminvorstufe und/oder eines derer Derivate enthalten. Dabei sind erfindungsgemäß solche Vitamine, Provitamine und Vitaminvorstufen bevorzugt, die üblicherweise den Gruppen A, B, C, E, F und H zugeordnet werden.

Weitere Pflegestoffe sind Panthenol, Coffein, Nicotinamid und Sorbitol.

Als Pflegestoff können die erfindungsgemäßen Mittel weiterhin mindestens einen Pflanzenextrakt, aber auch Mono- bzw. Oligosaccharide und/oder Lipide enthalten.

Die Zusammensetzung einiger besonders bevorzugter erfindungsgemäßer kosmetischer Zubereitungen kann den folgenden Tabellen entnommen werden (Angaben in Gew.-% bezogen auf das Gesamtgewicht des kosmetischen Mittels sofern nicht anders angegeben). Bezüglich weiterer bevorzugter Ausführungsformen dieser besonders bevorzugten Zusammensetzungen gilt mutatis mutandis das zuvor zu den erfindungsgemäßen kosmetischen Zubereitungen a) Gesagte.

| | Formel 1 | Formel 2 | Formel 3 | Formel 4 | Formel 5 |
|---|---|---|---|---|---|
| Ethanol | 20 bis 99 | 30 bis 90 | 40 bis 88 | 54 | 84 |
| Riechstoff a2) | 0,05 bis 20 | 0,1 bis 15 | 0,5 bis 10 | 0,7 | 2,3 |
| optionale Additive | add 100 | add 100 | add 100 | add 100 | add 100 |

| | Formel 6 | Formel 7 | Formel 8 | Formel 9 | Formel 10 |
|---|---|---|---|---|---|
| Ethanol und Wasser | 80 bis 99 | 90 bis 99 | 95 bis 99 | 98 | 96 |
| Riechstoff a2) | 0,05 bis 20 | 0,1 bis 15 | 0,5 bis 10 | 0,7 | 2,3 |
| optionale Additive | add 100 | add 100 | add 100 | add 100 | add 100 |

| | Formel 11 | Formel 12 | Formel 13 | Formel 14 | Formel 15 |
|---|---|---|---|---|---|
| Ethanol | 20 bis 99 | 30 bis 90 | 40 bis 88 | 54 | 84 |
| Riechstoff a2) | 0,05 bis 20 | 0,1 bis 15 | 0,5 bis 10 | 0,7 | 2,3 |
| nichtionisches Tensid a3) | 0,02 bis 4,0 | 0,05 bis 2,0 | 0,1 bis 1,0 | 0,4 | 0,1 |
| optionale Additive | add 100 | add 100 | add 100 | add 100 | add 100 |

| | Formel 16 | Formel 17 | Formel 18 | Formel 19 | Formel 20 |
|---|---|---|---|---|---|
| Ethanol und Wasser | 80 bis 99 | 90 bis 99 | 95 bis 99 | 98 | 96 |
| Riechstoff a2) | 0,05 bis 20 | 0,1 bis 15 | 0,5 bis 10 | 0,7 | 2,3 |
| nichtionisches Tensid a3) | 0,02 bis 4,0 | 0,05 bis 2,0 | 0,1 bis 1,0 | 0,4 | 0,1 |
| optionale Additive | add 100 | add 100 | add 100 | add 100 | add 100 |

| | Formel 21 | Formel 22 | Formel 23 | Formel 24 | Formel 25 |
|---|---|---|---|---|---|
| Ethanol | 20 bis 99 | 30 bis 90 | 40 bis 88 | 54 | 84 |
| Riechstoff a2) mit einem Siedepunkt >120°C | 0,05 bis 20 | 0,1 bis 15 | 0,5 bis 10 | 0,7 | 2,3 |
| optionale Additive | add 100 | add 100 | add 100 | add 100 | add 100 |

| | Formel 26 | Formel 27 | Formel 28 | Formel 29 | Formel 30 |
|---|---|---|---|---|---|
| Ethanol und Wasser | 80 bis 99 | 90 bis 99 | 95 bis 99 | 98 | 96 |
| Riechstoff a2) mit einem Siedepunkt >120°C | 0,05 bis 20 | 0,1 bis 15 | 0,5 bis 10 | 0,7 | 2,3 |
| optionale Additive | add 100 | add 100 | add 100 | add 100 | add 100 |

| | Formel 31 | Formel 32 | Formel 33 | Formel 34 | Formel 35 |
|---|---|---|---|---|---|
| Ethanol | 20 bis 99 | 30 bis 90 | 40 bis 88 | 54 | 84 |
| Riechstoff a2) mit einem Siedepunkt >120°C | 0,05 bis 20 | 0,1 bis 15 | 0,5 bis 10 | 0,7 | 2,3 |
| nichtionisches Tensid a3) | 0,02 bis 4,0 | 0,05 bis 2,0 | 0,1 bis 1,0 | 0,4 | 0,1 |
| optionale Additive | add 100 | add 100 | add 100 | add 100 | add 100 |

| | Formel 36 | Formel 37 | Formel 38 | Formel 39 | Formel 40 |
|---|---|---|---|---|---|
| Ethanol und Wasser | 80 bis 99 | 90 bis 99 | 95 bis 99 | 98 | 96 |
| Riechstoff a2) mit einem Siedepunkt >120°C | 0,05 bis 20 | 0,1 bis 15 | 0,5 bis 10 | 0,7 | 2,3 |
| nichtionisches Tensid a3) | 0,02 bis 4,0 | 0,05 bis 2,0 | 0,1 bis 1,0 | 0,4 | 0,1 |
| optionale Additive | add 100 | add 100 | add 100 | add 100 | add 100 |

Ganz besonders bevorzugte kosmetische Zubereitungen enthalten neben den zuvor beschriebenen Bestandteilen a1) bis a3) nur geringe Mengen weiterer Wirk- und Hilfsstoffe. Kosmetische Zubereitungen, dadurch gekennzeichnet, dass der Gewichtsanteil der Bestandteile a1), a2) sowie, falls enthalten, a3) am Gesamtgewicht der kosmetischen Zubereitung mindestens 80 Gew.-%, vorzugsweise mindestens 90 Gew.-%, besonders bevorzugt mindestens 93 Gew.-% und insbesondere mindestens 97 Gew.-% beträgt, sind aufgrund ihrer einfachen Herstellbarkeit und guten kosmetischen Wirkung besonders bevorzugt. Ganz besonders bevorzugte kosmetische Zubereitungen bestehen, bezogen auf ihr Gesamtgewicht, zu mindestens 80 Gew.-%, vorzugsweise mindestens 90 Gew.-% und insbesondere mindestens 97 Gew.-% aus den Bestanteilen a1) und a2).

Ganz besonders bevorzugte kosmetische Zubereitungen enthalten neben den zuvor beschriebenen Bestandteilen a1) bis a5) nur geringe Mengen weiterer Wirk- und Hilfsstoffe. Kosmetische Zubereitungen, dadurch gekennzeichnet, dass der Gewichtsanteil der Bestandteile a1), a2) sowie, falls enthalten, a3) und/oder a4) und/oder a5) am Gesamtgewicht der kosmetischen Zubereitung mindestens 80 Gew.-%, vorzugsweise mindestens 90 Gew.-%, besonders bevorzugt mindestens 93 Gew.-% und insbesondere mindestens 97 Gew.-% beträgt, sind aufgrund ihrer einfachen Herstellbarkeit und guten kosmetischen Wirkung besonders bevorzugt. Ganz besonders bevorzugte kosmetische Zubereitungen bestehen, bezogen auf ihr Gesamtgewicht, zu mindestens 80 Gew.-%, vorzugsweise mindestens 87 Gew.-% und insbesondere mindestens 95 Gew.-% aus den Bestanteilen a1), a2) und a3).

Wie eingangs ausgeführt eignen sich die erfindungsgemäßen kosmetischen Zubereitungen a) in besonderer Weise für die Applikation mittels einer Vorrichtung zur Entspannungsverdampfung. Ein weiterer Gegenstand der vorliegenden Anmeldung ist daher die Verwendung einer kosmetischen Zubereitung a) enthaltend, bezogen auf ihr Gesamtgewicht,
a1) 20 bis 99 Gew.-% Ethanol;
a2) 0,05 bis 20 Gew.-% Riechstoff;
als Prozessgut in einer Vorrichtung zur Entspannungsverdampfung.

Gegenstand der vorliegenden Anmeldung ist zudem die Verwendung eines erfindungsgemäßen Produkts zur Beaufschlagung menschlicher Haut oder menschlicher Haare, mit einer kosmetischen Zubereitung a) bzw. zur Beduftung menschlicher Haut oder menschlicher Haare.

Ein Verfahren zur Beduftung menschlicher Haut oder menschlicher Haare, bei welchem die keratinhaltigen Fasern mittels einer Vorrichtung zur Entspannungsverdampfung mit einer kosmetischen Zubereitung a) enthaltend bezogen auf ihr Gesamtgewicht
a1) 20 bis 99 Gew.-% Ethanol;
a2) 0,05 bis 20 Gew.-% Riechstoff;
beaufschlagt werden, ist ein weiterer Gegenstand der vorliegenden Anmeldung. Die kosmetische Zubereitung a) wird mittels der Vorrichtung zur Entspannungsverdampfung vorzugsweise in einen Sprühnebel überführt, welcher nachfolgend die keratinhaltigen Fasern beaufschlagt.

Um eine ausreichende Sprühwirkung zu erzielen, wird die kosmetische Zubereitung a) vorzugsweise auf Temperaturen oberhalb des Siedepunktes des in der kosmetischen Zubereitung a) enthaltenen polaren Lösungsmittels oder Lösungsmittelgemisches erhitzt.

Handelt es sich bei dem polaren Lösungsmittel um Wasser oder Lösungsmittelgemische mit einem Wasseranteil oberhalb 50 Gew.-% (bezogen auf das Gesamtgewicht des Lösungsmittelgemisches), wird die kosmetische Zubereitung vorzugsweise auf Temperaturen oberhalb 100°C, vorzugsweise auf Temperaturen von 100°C und 240°C, besonders bevorzugt auf Temperaturen von 140°C bis 160°C erhitzt.

Der durch die Erhitzung der kosmetischen Zubereitung a) erzielte Überdruck beträgt in den Fällen, in denen es sich bei dem polaren Lösungsmittel um Wasser oder Lösungsmittelgemische mit einem Wasseranteil oberhalb 50 Gew.-% (bezogen auf das Gesamtgewicht des Lösungsmittelgemisches) handelt, vorzugsweise zwischen 1,1 und 8 bar, bevorzugt zwischen 1,2 und 4 bar.

Ein bevorzugter Anmeldungsgegenstand ist ein Verfahren zur Farbveränderung keratinhaltiger Fasern, insbesondere menschlicher Haare, bei welchem die keratinhaltigen Fasern mittels einer Vorrichtung zur Entspannungsverdampfung mit einer kosmetischen Zubereitung a) enthaltend bezogen auf ihr Gesamtgewicht
a1) 20 bis 99 Gew.-% Ethanol;
a2) 0,05 bis 20 Gew.-% Riechstoff;
beaufschlagt werden, wobei
- aus einem Vorratsbehälter, in dessen Inneren ein Druck herrscht, der dem Umgebungsdruck entspricht, eine Teilmenge der in diesem Vorratsbehälter befindlichen kosmetischen Zubereitung a) in einen Behälter b1) überführt wird;
- nachfolgend der Zugang zwischen Vorratsbehälter und Behälter b1) durch ein Bauteil zur Durchflussregelung, mittels dessen der Durchfluss der kosmetischen Zubereitung a) aus dem Vorratsbehälter in den Behälter b1) unterbrochen werden kann, unterbrochen wird;
- nachfolgend die in dem gegen die Umgebung abgeschlossenen Behälter b1) befindliche kosmetische Zubereitung a) mittels einer Heizvorrichtung erhitzt wird, so dass der Druck im Inneren des Behälters b1) auf Werte oberhalb des Umgebungsdrucks, vorzugsweise auf Werte zwischen 1,1 und 8 bar, insbesondere auf Werte zwischen 1,2 und 4 bar ansteigt;
- nachfolgend der unter einem Druck oberhalb des Umgebungsdrucks stehende Behälter b1) in einer Weise geöffnet, wird, welche den Austritt mindestens einer Teilmenge, vorzugsweise mindestens 50 Gew.-%, bevorzugt mindestens 80 Gew.-% und insbesondere mindestens 90 Gew.-% der in dem Behälter b1) befindlichen kosmetischen Zubereitung aus dem Behälter b1) in die Umgebung unter Minderung des zum Zeitpunkt der Behälteröffnung in dem Behälter b1) herrschenden Drucks, entspannt wird.

Die Entspannung der kosmetischen Zubereitung a) in die Umgebung erfolgt vorzugsweise unter Ausbildung eines Sprühnebels der kosmetischen Zubereitung a).

Die aus dem Behälter b1) entspannte kosmetische Zubereitung a) wird vorzugsweise auf keratinische Fasern, insbesondere menschliche Haare aufgebracht.

Verfahren, in deren Verlauf die aus dem Behälter b1) entspannte kosmetische Zubereitung vor der Beaufschlagung der keratinischen Fasern durch eine Düse geleitet wird, sind erfindungsgemäß.

## Patentansprüche

1. Kosmetisches Produkt, umfassend
a) eine flüssige kosmetische Zubereitung, enthaltend bezogen auf ihr Gesamtgewicht
a1) 20 bis 99 Gew.-% Ethanol;
a2) 0,05 bis 20 Gew.-% Riechstoff;
b) eine Vorrichtung zur Entspannungsverdampfung der kosmetischen Zubereitung a) mit
b1) einem Behälter, welcher einen geschlossenen Innenraum definiert, in dem die kosmetische Zubereitung aufgenommen werden kann,
b2) einem Ventil oder einem vergleichbar wirkenden Schließelement, um den wenigstens anteilsweise mit der kosmetischen Zubereitung a) befüllten Innenraum des Behälters zu verschließen und zu öffnen,
b3) einer Heizvorrichtung, um die in dem geschlossenen Innenraum des Behälters befindliche kosmetische Zubereitung a) auf Temperaturen oberhalb der Umgebungstemperatur in einer Weise zu erhitzen, dass in dem Behälter ein Druck oberhalb des Umgebungsdruck entsteht, sowie die erhitzte und unter erhöhtem Druck stehende kosmetische Zubereitung a) aus dem Innenraum des Behälters unter Druckminderung in die Umgebung zu entspannen,
b4) einer Düse, welche eine Zerstäubung der aus dem Behälter entweichenden kosmetischen Zubereitung a) ermöglicht.

2. Kosmetisches Produkt nach Anspruch 1, **dadurch gekennzeichnet, dass** der Gewichtsanteil des Ethanols a1) am Gesamtgewicht der kosmetischen Zubereitung a) 30 bis 90 Gew.-%, vorzugsweise 40 bis 80 Gew.-% beträgt.

3. Kosmetisches Produkt nach einem der vorherigen Ansprüche, **dadurch gekennzeichnet, dass** der Gewichtsanteil des Riechstoffs am Gesamtgewicht der kosmetischen Zubereitung a) 0,1 bis 15 Gew.-% und insbesondere 0,5 bis 10 Gew.-% beträgt.

4. Kosmetisches Produkt nach einem der vorherigen Ansprüche, **dadurch gekennzeichnet, dass** der Riechstoff a2) einen Siedepunkt oberhalb 80°C, vorzugsweise oberhalb 120°C und insbesondere oberhalb 160°C aufweist.

5. Kosmetisches Produkt nach einem der vorherigen Ansprüche, **dadurch gekennzeichnet, dass** die kosmetische Zubereitung a) bezogen auf ihr Gesamtgewicht 0,02 bis 4,0 Gew.-%, vorzugsweise 0,05 bis 2,0 Gew.-% und insbesondere 0,1 bis 1,0 Gew.-% nichtionisches Tensid a3) enthält.

6. Kosmetisches Produkt nach einem der vorherigen Ansprüche, **dadurch gekennzeichnet, dass** die kosmetische Zubereitung a) bezogen auf ihr Gesamtgewicht, zu mindestens 80 Gew.-%, vorzugsweise mindestens 90 Gew.-% und insbesondere mindestens 97 Gew.-% aus den Bestanteilen a1) und a2) besteht.

7. Verwendung einer flüssigen kosmetischen Zubereitung a) enthaltend, bezogen auf ihr Gesamtgewicht,
a1) 20 bis 99 Gew.-% Ethanol;
a2) 0,05 bis 20 Gew.-% Riechstoff;
als Prozessgut in einer Vorrichtung zur Entspannungsverdampfung der kosmetischen Zubereitung a) mit
b1) einem Behälter, welcher einen geschlossenen Innenraum definiert, in dem die kosmetische Zubereitung aufgenommen werden kann,
b2) einem Ventil oder einem vergleichbar wirkenden Schließelement, um den wenigstens anteilsweise mit der kosmetischen Zubereitung a) befüllten Innenraum des Behälters zu verschließen und zu öffnen,
b3) einer Heizvorrichtung, um die in dem geschlossenen Innenraum des Behälters befindliche kosmetische Zubereitung a) auf Temperaturen oberhalb der Umgebungstemperatur in einer Weise zu erhitzen, dass in dem Behälter ein Druck oberhalb des Umgebungsdruck entsteht, sowie die erhitzte und unter erhöhtem Druck stehende kosmetische Zubereitung a) aus dem Innenraum des Behälters unter Druckminderung in die Umgebung zu entspannen,
b4) einer Düse, welche eine Zerstäubung der aus dem Behälter entweichenden kosmetischen Zubereitung a) ermöglicht.

8. Verwendung eines Produkts nach einem der Ansprüche 1 bis 6 zur Beaufschlagung menschlicher Haut oder menschlicher Haare, mit einer kosmetischen Zubereitung a).

9. Verwendung eines Produkts nach einem der Ansprüche 1 bis 6 zur Beduftung menschlicher Haut oder menschlicher Haare.

10. Verfahren zur Beduftung menschlicher Haut oder menschlicher Haare, bei welchem die Haut oder die Haare mittels einer Vorrichtung zur Entspannungsverdampfung einer kosmetischen Zubereitung mit
b1) einem Behälter, welcher einen geschlossenen Innenraum definiert, in dem die kosmetische Zubereitung aufgenommen werden kann,
b2) einem Ventil oder einem vergleichbar wirkenden Schließelement, um den wenigstens anteilsweise mit der kosmetischen Zubereitung a) befüllten Innenraum des Behälters zu verschließen und zu öffnen,
b3) einer Heizvorrichtung, um die in dem geschlossenen Innenraum des Behälters befindliche kosmetische Zubereitung a) auf Temperaturen oberhalb der Umgebungstemperatur in einer Weise zu erhitzen, dass in dem Behälter ein Druck oberhalb des Umgebungsdruck entsteht, sowie die erhitzte und unter erhöhtem Druck stehende kosmetische Zubereitung a) aus dem Innenraum des Behälters unter Druckminderung in die Umgebung zu entspannen,
b4) einer Düse, welche eine Zerstäubung der aus dem Behälter entweichenden kosmetischen Zubereitung a) ermöglicht,
mit einer flüssigen kosmetischen Zubereitung a) enthaltend bezogen auf ihr Gesamtgewicht
a1) 20 bis 99 Gew.-% Ethanol;
a2) 0,05 bis 20 Gew.-% Riechstoff;
beaufschlagt werden.

11. Verfahren nach Anspruch 10, **dadurch gekennzeichnet, dass**
- aus einem Vorratsbehälter, in dessen Inneren ein Druck herrscht, der dem Umgebungsdruck entspricht, eine Teilmenge der in diesem Vorratsbehälter befindlichen kosmetischen Zubereitung a) in einen Behälter überführt wird;
- nachfolgend der Zugang zwischen Vorratsbehälter und Behälter durch ein Bauteil zur Durchflussregelung, mittels dessen der Durchfluss der kosmetischen Zubereitung a) aus dem Vorratsbehälter in den Behälter unterbrochen werden kann, unterbrochen wird;
- nachfolgend die in dem gegen die Umgebung abgeschlossenen Behälter befindliche kosmetische Zubereitung a) mittels einer Heizvorrichtung erhitzt wird, so dass der Druck im Inneren des Behälters auf Werte oberhalb des Umgebungsdrucks, vorzugsweise auf Werte zwischen 1,1 und 8 bar, insbesondere auf Werte zwischen 1,2 und 4 bar ansteigt;
- nachfolgend der unter einem Druck oberhalb des Umgebungsdrucks stehende Behälter in einer Weise geöffnet, wird, welche den Austritt mindestens einer Teilmenge, vorzugsweise mindestens 50 Gew.-%, bevorzugt mindestens 80 Gew.-% und insbesondere mindestens 90 Gew.-% der in dem Behälter befindlichen kosmetischen Zubereitung aus dem Behälter in die Umgebung unter Minderung des zum Zeitpunkt der Behälteröffnung in dem Behälter herrschenden Drucks, entspannt wird.

## Claims

1. A cosmetic product comprising
a) a liquid cosmetic preparation containing, based on the total weight thereof,
a1) 20 to 99 wt.% ethanol;
a1) 0.05 to 20 wt.% odorant;
b) a device for flash evaporation of the cosmetic preparation a), comprising b1)
a container, which defines a closed inner chamber in which the cosmetic preparation can be stored,
b2) a valve or a similarly functioning closing element, in order to close and open the inner chamber of the container filled at least in part with the cosmetic preparation a),
b3) a heating device, in order to heat the cosmetic preparation a) in the closed interior of the container to temperatures above ambient temperature in such a way that a pressure above the ambient pressure is produced in the container, and to pressure-relieve the heated and pressurized cosmetic preparation a) from the inner chamber of the container into the surrounding region by lowering the pressure,
b4) a nozzle, which makes it possible to atomize the cosmetic preparation a) escaping from the container.

2. The cosmetic product according to claim 1, **characterized in that** the weight proportion of the ethanol a1) with respect to the total weight of the cosmetic preparation a) is 30 to 90 wt.%, preferably 40 to 80 wt.%.

3. The cosmetic product according to one of the preceding claims, **characterized in that** the weight proportion of the odorant with respect to the total weight of the cosmetic preparation a) is 0.1 to 15 wt.% and in particular 0.5 to 10 wt.%.

4. The cosmetic product according to one of the preceding claims, **characterized in that** the odorant a2) has a boiling point above 80 °C, preferably above 120 °C and in particular above 160 °C.

5. The cosmetic product according to one of the preceding claims, **characterized in that** the cosmetic preparation a) contains, based on its total weight, 0.02 to 4.0 wt.%, preferably 0.05 to 2.0 wt.% and in particular 0.1 to 1.0 wt.%, non-ionic surfactant a3).

6. The cosmetic product according to one of the preceding claims, **characterized in that** at least 80 wt.%, preferably at least 90 wt.% and in particular at least 97 wt.%, of the cosmetic preparation a), based on its total weight, consists of the components a1) and a2).

7. The use of a liquid cosmetic preparation a) containing, based on its total weight,
a1) 20 to 99 wt.% ethanol;
a2) 0.05 to 20 wt.% odorant;
as a process material in a device for flash evaporation of the cosmetic preparation a), comprising
b1) a container, which defines a closed inner chamber in which the cosmetic preparation can be stored,
b2) a valve or a similarly functioning closing element, in order to close and open the inner chamber of the container filled at least in part with the cosmetic preparation a),
b3) a heating device, in order to heat the cosmetic preparation a) in the closed interior of the container to temperatures above ambient temperature in such a way that a pressure above the ambient pressure is produced in the container, and to pressure-relieve the heated and pressurized cosmetic preparation a) from the inner chamber of the container into the surrounding region by lowering the pressure,
b4) a nozzle, which makes it possible to atomize the cosmetic preparation a) escaping from the container.

8. The use of a product according to one of claims 1 to 6 for applying a cosmetic preparation a) to human skin or human hair.

9. The use of a product according to one of claims 1 to 6 for fragrancing human skin or human hair.

10. A method for fragrancing human skin or human hair, in which the skin or the hair, by means of a device for flash evaporation of a cosmetic preparation comprising
b1) a container, which defines a closed inner chamber in which the cosmetic preparation can be stored,
b2) a valve or a similarly functioning closing element, in order to close and open the inner chamber of the container filled at least in part with the cosmetic preparation a),
b3) a heating device, in order to heat the cosmetic preparation a) in the closed interior of the container to temperatures above ambient temperature in such a way that a pressure above the ambient pressure is produced in the container, and to pressure-relieve the heated and pressurized cosmetic preparation a) from the inner chamber of the container into the surrounding region by lowering the pressure,
b4) a nozzle, which makes it possible to atomize the cosmetic preparation a) escaping from the container,
is subjected to a liquid cosmetic preparation a) containing, based on its total weight,
a1) 20 to 99 wt.% ethanol;
a2) 0.05 to 20 wt.% odorant.

11. The method according to claim 10, **characterized in that**
- some of the cosmetic preparation a) in a reservoir, inside which reservoir a pressure corresponding to the ambient pressure prevails, is transferred from said reservoir into a container;
- using a component for controlling flow, by means of which the flow of the cosmetic preparation a) from the reservoir into the container can be interrupted, access between the reservoir and the container is subsequently interrupted;
- the cosmetic preparation a) in the container, which is sealed against the surrounding region, is subsequently heated by means of a heating device, such that the pressure inside the container increases to values above the ambient pressure, preferably to values between 1.1 and 8 bar, in particular to values between 1.2 and 4 bar;
- the container, which is pressurized above the ambient pressure, is subsequently opened in such a way that the discharge of at least some, preferably at least 50 wt.%, more preferably at least 80 wt.%, and in particular at least 90 wt.%, of the cosmetic preparation in the container is pressure-relieved from the container into the surrounding region by lowering the pressure prevailing in the container at the time at which the container is opened.

## Revendications

1. Produit cosmétique, comprenant
a) une préparation cosmétique liquide contenant, par rapport à son poids total,
a1) 20 à 99 % en poids d'éthanol ;
a2) 0,05 à 20 % en poids de parfum ;
b) un dispositif de vaporisation par détente de la préparation cosmétique a) comportant b1)
un récipient définissant un espace intérieur fermé dans lequel la préparation cosmétique peut être reçue,
b2) une soupape ou un élément de fermeture à action comparable permettant de fermer et d'ouvrir l'espace intérieur du récipient rempli au moins partiellement de préparation cosmétique a),
b3) un dispositif de chauffage permettant de chauffer la préparation cosmétique a) se trouvant dans l'espace intérieur fermé du récipient à des températures supérieures à la température ambiante, de sorte qu'une pression supérieure à la pression ambiante soit produite dans le récipient, et de détendre la préparation cosmétique a) chauffée et sous pression élevée hors de l'espace intérieur du récipient tout en réduisant la pression dans l'environnement,
b4) une buse permettant de vaporiser la préparation cosmétique a) s'échappant du récipient

2. Produit cosmétique selon la revendication 1, **caractérisé en ce que** la proportion en poids de l'éthanol a1) dans le poids total de la préparation cosmétique a) est comprise entre 30 et 90 % en poids, de préférence entre 40 et 80 % en poids.

3. Produit cosmétique selon l'une des revendications précédentes, **caractérisé en ce que** la proportion en poids du parfum dans le poids total de la préparation cosmétique a) est comprise entre 0,1 et 15 % en poids et en particulier entre 0,5 et 10 % en poids.

4. Produit cosmétique selon l'une des revendications précédentes, **caractérisé en ce que** le parfum a2) présente un point d'ébullition supérieur à 80 °C, de préférence supérieur à 120 °C et en particulier à 160 °C.

5. Produit cosmétique selon l'une des revendications précédentes, **caractérisé en ce que** la préparation cosmétique a) contient, par rapport à son poids total, 0,02 à 4,0 % en poids, de préférence 0,05 à 2,0 % en poids et en particulier 0,1 à 1,0 % en poids de tensioactif non ionique a3).

6. Produit cosmétique selon l'une des revendications précédentes, **caractérisé en ce que** la préparation cosmétique a) est constituée, par rapport à son poids total, d'au moins 80 % en poids, de préférence d'au moins 90 % en poids et en particulier d'au moins 97 % en poids des constituants a1) et a2).

7. Utilisation d'une préparation cosmétique a) liquide contenant, par rapport à son poids total,
a1) 20 à 99 % en poids d'éthanol ;
a2) 0,05 à 20 % en poids de parfum ;
comme produit de traitement dans un dispositif de vaporisation par détente de la préparation cosmétique a) comportant
b1) un récipient définissant un espace intérieur fermé dans lequel la préparation cosmétique peut être reçue,
b2) une soupape ou un élément de fermeture à action comparable permettant de fermer et d'ouvrir l'espace intérieur du récipient rempli au moins partiellement de préparation cosmétique a),
b3) un dispositif de chauffage permettant de chauffer la préparation cosmétique a) se trouvant dans l'espace intérieur fermé du récipient à des températures supérieures à la température ambiante, de sorte qu'une pression supérieure à la pression ambiante soit produite dans le récipient, et de détendre la préparation cosmétique a) chauffée et sous pression élevée hors de l'espace intérieur du récipient tout en réduisant la pression dans l'environnement,
b4) une buse permettant de vaporiser la préparation cosmétique a) s'échappant du récipient.

8. Utilisation d'un produit selon l'une des revendications 1 à 6 pour l'application d'une préparation cosmétique a) sur la peau ou les cheveux humains.

9. Utilisation d'un produit selon l'une des revendications 1 à 6 pour parfumer la peau ou les cheveux humains.

10. Procédé destiné à parfumer la peau ou les cheveux humains, dans lequel la peau ou les cheveux humains sont parfumés au moyen d'un dispositif de vaporisation par détente d'une préparation cosmétique contenant
b1) un récipient définissant un espace intérieur fermé dans lequel la préparation cosmétique peut être reçue,
b2) une soupape ou un élément de fermeture à action comparable permettant de fermer et d'ouvrir l'espace intérieur du récipient rempli au moins partiellement de préparation cosmétique a),
b3) un dispositif de chauffage permettant de chauffer la préparation cosmétique a) se trouvant dans l'espace intérieur fermé du récipient à des températures supérieures à la température ambiante, de sorte qu'une pression supérieure à la pression ambiante soit produite dans le récipient, et de détendre la préparation cosmétique a) chauffée et sous pression élevée hors de l'espace intérieur du récipient tout en réduisant la pression dans l'environnement,
b4) une buse permettant de vaporiser la préparation cosmétique a) s'échappant du récipient,
une préparation cosmétique a) liquide contenant, par rapport à son poids total,
a1) 20 à 99 % en poids d'éthanol ;
a2) 0,05 à 20 % en poids de parfum.

11. Procédé selon la revendication 10, **caractérisé en ce que**,
- à partir d'un réservoir de stockage à l'intérieur duquel règne une pression égale à la pression ambiante, une fraction de la préparation cosmétique a) présente dans ce réservoir de stockage est transférée dans un récipient ;
- on interrompt ensuite l'accès entre le réservoir de stockage et le récipient par un élément de régulation de flux au moyen duquel le flux de la préparation cosmétique a) depuis le réservoir de stockage jusqu'au récipient peut être interrompu ;
- on chauffe ensuite la préparation cosmétique a) présente dans le récipient isolé de l'environnement au moyen d'un dispositif de chauffage, de sorte que la pression à l'intérieur du récipient dépasse la pression ambiante et atteint de préférence des valeurs comprises entre 1,1 et 8 bar, en particulier entre 1,2 et 4 bars ;
- le récipient se trouvant à une pression supérieure à la pression ambiante est ensuite ouvert de manière à détendre au moins une fraction, de préférence au moins 50 % en poids, de façon préférée au moins 80 % en poids et en particulier au moins 90 % en poids de la préparation cosmétique présente dans le récipient et à provoquer sa sortie dans l'atmosphère tout en réduisant la pression régnant dans le récipient au moment de l'ouverture de celui-ci.
